# EUROPÄISCHE PATENTSCHRIFT

(11) **EP 1 523 363 B1**
(45) Veröffentlichungstag und Bekanntmachung des Hinweises auf die Patenterteilung: **01.04.2009**
(21) Anmeldenummer: 03765037.1
(22) Anmeldetag: 18.07.2003
(51) Int. Cl.: A61M 13/00, A61M 11/04, A61M 11/06, A61M 19/00

(54) **VORRICHTUNG ZUR AUFBEREITUNG EINES INSUFFLATIONSGASES**
DEVICE FOR CONDITIONING AN INSUFFLATION GAS
DISPOSITIF DE PREPARATION D'UN GAZ D'INSUFFLATION

(30) Priorität: 19.07.2002 DE 10233860
(43) Veröffentlichungstag der Anmeldung: 20.04.2005
(73) Patentinhaber: Storz Endoskop Produktions GmbH, 78532 Tuttlingen (DE)
(72) Erfinder: DIEMUNSCH, Pierre, F-67000 Strasbourg (FR)
(74) Vertreter: Weller, Wolfgang
(86) Internationale Anmeldenummer: PCT/EP2003/007808
(87) Internationale Veröffentlichungsnummer: WO 2004/009166

(56) Entgegenhaltungen:
- EP-A- 0 937 478
- DE-A- 19 510 710
- DE-U- 29 612 115
- US-A- 5 246 419
- US-A- 5 599 297
- US-A- 6 010 118

## Beschreibung

Die Erfindung betrifft eine Vorrichtung zur Aufbereitung eines Insufflationsgases mit einem Einlass zum Zuführen eines Insufflationsgases, mit einer Befeuchtungsvorrichtung zur Befeuchtung des Insufflationsgases mit einer Befeuchtungsflüssigkeit, die ein Arzneimittel enthält, und mit einem Auslass zum Abführen des mit dem Arzneimittel beladenen Insufflationsgases, wobei die Befeuchtungsvorrichtung eine Aufbereitungskammer aufweist, und wobei die Aufbereitungskammer zumindest eine Leitung zur Zufuhr eines in der Befeuchtungsflüssigkeit enthaltenen Arzneimittels aufweist.

Eine solche Vorrichtung ist beispielsweise aus der US 6,010,118 bekannt.

Eine wesentliche Grundlage für eine endoskopische Betrachtung des abdominalen Raumes eines Menschen oder eines Tieres stellt die Schaffung eines mit Gas aufgeblähten Raumes in der jeweiligen Bauchhöhle dar. In die Bauchhöhle wird mittels eines Insufflators ein Insufflationsgas gepumpt, wobei die Bauchhöhle sozusagen aufgebläht wird. Jedenfalls schafft das insufflierte Gas den für eine Untersuchung, ggf. für eine Operation, erforderlichen Betrachtungs- bzw. Operationsraum zwischen den inneren Organen und der Bauchdecke. Als Insufflationsgas verwendet man üblicherweise Kohlensäuregas.

Eine Vorrichtung zum Einbringen von Insufflationsgas in den abdominalen Raum ist bspw. aus dem Artikel "Die Laparoskopie in der Gynäkologie" von K. Semm in "Geburtshilfe und Frauenheilkunde", Heft 11, November 1967, bekannt. Mit dieser Vorrichtung können die Menge und der Druck des über eine Insufflationskanüle in den abdominalen Raum einströmenden Insufflationsgases gesteuert und überwacht werden.

Zur Vermeidung postoperativer Schmerzen und Komplikationen, zumindest aber um diese zu reduzieren, befeuchtet man das Insufflationsgas. Manchmal wird hierzu das Insufflationsgas auch erwärmt. Dabei hat sich herausgestellt, daß es im wesentlichen auf eine Befeuchtung des Insufflationsgases ankommt. Eine zusätzliche Aufwärmung des Insufflationsgases bringt demgegenüber keinen wesentlichen Zusatznutzen. Dies ist bspw. aus der Veröffentlichung "Central body temperature during prolonged laparoscopy decreases despite heating of the insufflated CO₂" von P. Diemunsch, D. Multer, R. Schaeffer, D. Graff, F. Hirezi und J. Marescaux bekannt.

Im Zusammenhang mit endoskopischen Untersuchungen ist es in vielen Fällen notwendig oder wünschenswert, dem Patienten Arzneimittel zu verabreichen, die antibakteriell, entzündungshemmend lokalanästhesierend oder antikarzinogen wirken. Diese Arzneimittel können bspw. systemisch über die Blutbahn verabreicht werden. Dies hat jedoch den Nachteil, daß die Arzneimittelgabe nicht auf den Bereich des Abdomens beschränkt ist, sondern im ganzen Körper stattfindet. Eine andere Möglichkeit zur Arzneimittelverabreichung besteht darin, das Arzneimittel als Beimischung zu einer Spülflüssigkeit in das Abdomen einzubringen. Zwar ist die Arzneimittelgabe dann auf das Abdomen beschränkt, allerdings werden nur die von der Spülflüssigkeit umspülten Gewebeflächen erreicht. Zudem ist es häufig nicht erforderlich, daß eine Spülflüssigkeit eingesetzt wird.

Die US 6,010,118 beschreibt einen medizinischen Gasbefeuchter zur Befeuchtung eines Insufflationsgases. Dieser Gasbefeuchter weist eine Befeuchtungskammer auf, in der ein wasserabsorbierendes Medium angeordnet ist, um das zumindest zum Teil ein Heizelement angeordnet ist. Das wasserabsorbierende Medium wird mit Wasser beladen und das zu befeuchtende Gas dann an dem beladenen wasserabsorbierenden Medium vorbeigeführt, wobei es geringe Mengen an Wasser verdampft und aufnimmt. Diese Aufnahme von Wasser wird noch dadurch erleichtert, daß das Wasser durch das Heizelement erwärmt wird. Es wird ferner offenbart, daß es das Design ermöglicht, der Befeuchtungskammer andere Substanzen als reines Wasser zuzugeben.

Die DE 296 12 115 U1 beschreibt eine Vorrichtung zur Befeuchtung von Insufflationsgas_{.} Das Insufflationsgas wird dadurch durch eine Kammer geleitet, in der Wasser zerstäubt wird. Das Insufflationsgas nimmt Teile des zerstäubten Wassers auf und wird dadurch befeuchtet. Die Kammer zeichnet sich dadurch aus, dass eine Membran vorliegt, welche lösbar mit einer externen energieübertragenden Einheit verbunden sein kann, die die Membran in Schwingung versetzt und dadurch das Wasser zerstäubt.

Die US 5,599,297 beschreibt einen Insufflator, der eine Vorrichtung zum Beladen des Insufflationsgases mit einem Arzneimittel aufweist. Bei dieser Vorrichtung zum Beladen handelt es sich um eine Vorrichtung zum Zerstäuben des Medikaments.

Die EP 0 937 478 A1 beschreibt einen Insufflator, der eine Vorrichtung zur Arzneimittelgabe mittels eines Insufflationsgases aufweist. Die in diesem Dokument beschriebene Vorrichtung zur Arzneimittelgabe weist dabei eine Mikropumpe zum Einbringen von Medikamenten in den Insufflationsgasstrom auf.

Die US 5,246,419 beschreibt einen Insufflator, der Mittel zur Zugabe von Arzneimitteln zu einem Insufflationsgasstrom aufweist. Dies erfolgt in diesem Dokument mit Hilfe eines Verneblers.

Die DE 195 10 710 A1 beschreibt eine Vorrichtung zum Konditionieren von Insufflationsgas. Diese Vorrichtung dient zum Befeuchten bzw. Temperieren des Insufflationsgases.

Es ist daher Aufgabe der vorliegenden Erfindung, im Zusammenhang mit einem medizinischen Eingriff, bei dem der abdominale Raum eines Menschen oder Tieres mit einem Insufflationsgas gefüllt wird, die Verabreichung eines oder mehrerer Arzneimittel zu ermöglichen.

Die Aufgabe wird erfindungsgemäß dadurch gelöst, dass in einer Vorrichtung zur Aufbereitung eines Insufflationsgases die Befeuchtungsvorrichtung eine Einblasvorrichtung zum Einblasen des Insufflationsgases in die das Arzneimittel enthaltende Befeuchtungsflüssigkeit aufweist, aus der das Insufflationsgas zu dem Auslass hin unter Mitnahme von das Arzneimittel enthaltender Befeuchtungsflüssigkeit austreten kann, und dass die Vorrichtung zur Aufbereitung eines Insufflationsgases eine Dosiervorrichtung zur dosierbaren Zufuhr des Arzneimittels zu der Befeuchtungsflüssigkeit aufweist.

Erfindungsgemäß kommt dem Insufflationsgas somit eine Mehrfachfunktion zu. Es dient in an sich bekannter Weise zur Bildung eines aufgeblähten Raumes im abdominalen Bereich eines Menschen oder Tieres, so daß dort für eine endoskopische Maßnahme der erforderliche Betrachtungsraum geschaffen wird. Zudem dient das Insufflationsgas als Träger für mindestens ein Arzneimittel. Das Insufflationsgas erreicht auch verzweigte und enge Bereiche des abdominalen Bereiches und sorgt somit für eine optimale Verteilung des Arzneimittels, mit dem es beladen ist. Dabei diffundiert das Insufflationsgas, unterstützt durch den Druck, unter dem es steht, auch in eng verzweigte Gewebeteile. Die Arzneimittelgabe erfolgt lokal im abdominalen Bereich. Dadurch können gezielt, effektiv und mit möglichst geringer Dosierung ein oder mehrere Arzneimittel verabreicht werden. Dadurch können auch Nebenwirkungen verringert oder sogar völlig vermieden werden. Eine Arzneimittelgabe, bspw. systemisch über die Blutbahn ist dann nicht mehr erforderlich, was die Behandlung sehr vereinfacht.

Bei dem Arzneimittel kann es sich bspw. um antibakterielle Arzneimittel, entzündungshemmende Arzneimittel, Lokalanästhetika oder antikarzinogene Arzneimittel handeln. Es versteht sich, daß das Insufflationsgas in erfindungsgemäßer Weise auch mit Wirkstoffkombinationen beladbar ist.

In einer Ausgestaltung der Erfindung weist die erfindungsgemäße Vorrichtung Temperiermittel zum Temperieren der Befeuchtungsflüssigkeit auf.

Mit Hilfe der Temperiermittel, die zum Abkühlen und/oder zum Erwärmen der Befeuchtungsflüssigkeit ausgestaltet sein können, kann diese optimal an das im Abdomen herrschende Mikroklima angepaßt werden. Insbesondere im Zusammenhang mit der obengenannten Maßnahme, bei der das Insufflationsgas an der Befeuchtungsflüssigkeit vorbeigeführt wird, ist eine Aufwärmung der Befeuchtungsflüssigkeit mit Hilfe der Temperiermittel bevorzugt. Ein Erwärmen einer Flüssigkeit führt zu einer Erhöhung des über dem Flüssigkeitsniveau herrschenden Dampfdruckes. Ein erhöhter Dampfdruck führt zu einer erhöhten Menge verdampfter Flüssigkeit und stellt ein Mittel zur Steuerung des Beladungsvorganges dar.

In diesem Zusammenhang ist es besonders vorteilhaft, wenn die Temperiermittel Heizmittel zum Verdampfen der Befeuchtungsflüssigkeit enthalten.

Auf diese Weise kann das Insufflationsgas mit einer besonders großen Menge von Befeuchtungsflüssigkeit beladen werden. Wenn die Befeuchtungsflüssigkeit bspw. Wasser ist, kann das Insufflationsgas durch den mit Hilfe der Heizmittel erzeugten Wasserdampf gesättigt werden.

Die im folgenden beschriebenen weiteren Ausgestaltungen der Erfindung betreffen die Aufbereitung der Befeuchtungsflüssigkeit mit dem Arzneimittel.

In einer weiteren Ausgestaltung der Erfindung weist die erfindungsgemäße Vorrichtung Verteilmittel zur Verteilung des Arzneimittels in der Befeuchtungsflüssigkeit auf.

Die Verteilmittel verteilen Arzneimittel, die in der Befeuchtungsflüssigkeit nicht löslich sind, bspw. hydrophob sind, gleichmäßig in der Befeuchtungsflüssigkeit, wobei bspw. eine Emulsion oder Aufschlämmung gebildet wird. Diese kann dann von der Befeuchtungsvorrichtung in das Insufflationsgas überbracht werden.

In vorteilhaften Ausgestaltungen der Erfindung im Sinne der vorangegangenen Ausführung enthalten die Verteilmittel eine Rührvorrichtung zum Rühren und/oder eine Umwälzpumpe zum Umwälzen der Befeuchtungsflüssigkeit und des Arzneimittels.

Eine Rührvorrichtung sowie eine Umwälzpumpe sind konstruktiv einfach zu realisieren und wirkungsvoll.

In einer weiteren Ausgestaltung der Erfindung weist die erfindungsgemäße Vorrichtung Dosier-Steuermittel zur Dosierung des Anteils der das Arzneimittel enthaltenden Befeuchtungsflüssigkeit in dem Insufflationsgas auf.

Damit kann die medizinisch jeweils notwendige Menge des Arzneimittels in dem Insufflationsgas auf einfache Weise eingestellt werden.

In einer weiteren Ausgestaltung weist die Vorrichtung Gas-Steuermittel zur Steuerung der Menge und/oder des Drucks des dem Auslaß zugeführten Insufflationsgases auf.

Dadurch wird sozusagen eine integrierte Lösung geschaffen, mit der nicht nur ein Arzneimittel in das Abdomen eingebracht werden kann, sondern auch die Menge und der Druck des dem Abdomen zugeführten Insufflationsgases einstellbar ist. Die erfindungsgemäße Vorrichtung bildet dabei einen Insufflator. Ein separater Insufflator zur Einstellung von Druck und Menge des dem Abdominum zugeführten Insufflationsgases ist nicht erforderlich.

In einer weiteren Ausgestaltung weist die Vorrichtung eine Insufflationskanüle auf.

Mit Hilfe der Insufflationskanüle, bspw. einer Insufflationsnadel, ist die erfindungsgemäße Vorrichtung unmittelbar mit dem jeweils zu behandelnden Abdomen verbindbar.

In einer weiteren Ausgestaltung weist die Vorrichtung ein Anschlußstück für einen auswechselbaren Vorratsbehälter auf, der zur Aufnahme der das Arzneimittel enthaltenden Befeuchtungsflüssigkeit vorgesehen ist.

Für eine derartig ausgestaltete Vorrichtung ist ein Vorratsbehälter mit einem Anschlußgegenstück, mit dem der Vorratsbehälter mit dem Anschlußstück der Vorrichtung verbindbar ist, und mit einer ein Arzneimittel enthaltenden Befeuchtungsflüssigkeit vorgesehen. Der Vorratsbehälter bildet vorteilhaft einen Bestandteil der erfindungsgemäßen Vorrichtung.

Der Vorratsbehälter ist bspw. eine Art Kartusche. Ein entleerter Vorratsbehälter kann sehr einfach gegen einen befüllten Vorratsbehälter ausgetauscht werden. Zudem ist es leicht möglich, einen Vorratsbehälter mit einer Befeuchtungsflüssigkeit, in der ein erstes Arzneimittel gelöst ist, gegen einen anderen Vorratsbehälter auszutauschen, in dem ein zweites Arzneimittel gelöst ist.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind.

Die Erfindung wird nachfolgend anhand eines ausgewählten Ausführungsbeispiels im Zusammenhang mit den beiliegenden Zeichnungen näher beschrieben und erläutert. Es zeigt:

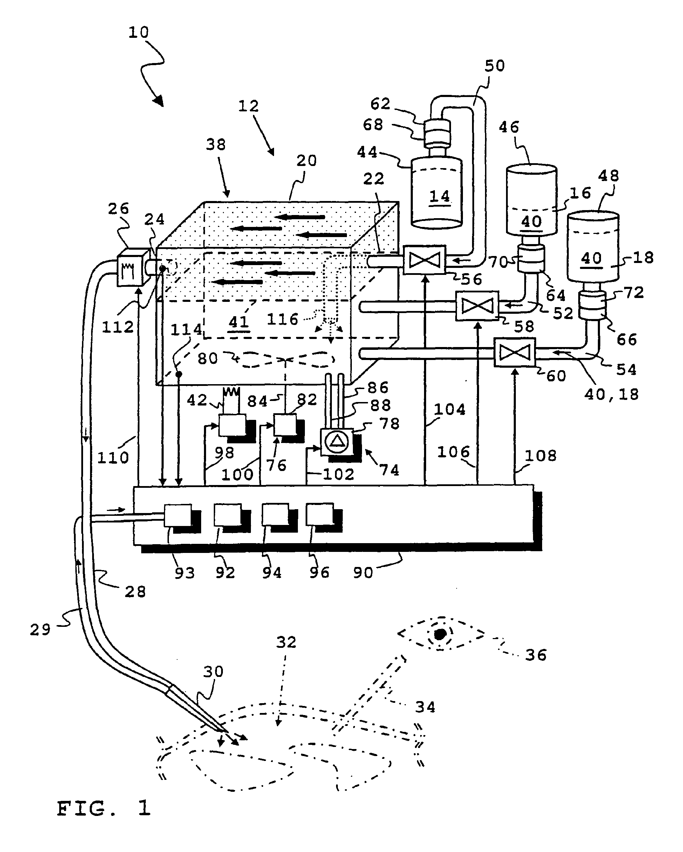

- Fig. 1: teilweise stark schematisiert eine perspektivische Ansicht einer Vorrichtung zur Aufbereitung eines Insufflationsgases, wobei zusätzlich in gepunkteten Linien eine Variante der Vorrichtung eingezeichnet ist.

In Fig. 1 stellt lediglich die in gepunkteten Linien dargestellte Variante eine Vorrichtung gemäß der Erfindung dar.

In Fig. 1 ist eine Vorrichtung zur Aufbereitung eines Insufflationsgases in ihrer Gesamtheit mit der Bezugsziffer 10 versehen. Die Vorrichtung 10 bildet einen Insufflator, mit dem ein Insufflationsgas einem abdominalen Raum eines Menschen oder Tieres zugeführt werden kann.

Die Vorrichtung 10 weist eine Beladungsvorrichtung 12 zur Beladung eines Insufflationsgases 14 mit Arzneimitteln 16 und 18 auf. Die Beladungsvorrichtung 12 weist eine Mitnahmevorrichtung 13 auf, in der ein Strom des Insufflationsgases 14 an den Arzneimitteln 16, 18 vorbeigeführt wird und dabei Partikel der Arzneimittel 16, 18 mitnimmt.

Die Mitnahmevorrichtung 13 enthält eine Aufbereitungskammer 20, in die das Insufflationsgas 14 über einen Einlaß 22 einströmen kann. Aus der Aufbereitungskammer 20 kann das mit den Arzneimitteln 16, 18 beladene Insufflationsgas 14 über einen Auslaß 24 ausströmen. Am Auslaß 24 ist eine Heizung 26 zum Erwärmen des Insufflationsgases 14 angeordnet. Von der Heizung 26 kann das mit den Arzneimitteln 16, 18 beladene Insufflationsgas 14 über eine vorliegend als Schlauchleitung ausgeführte Leitung 28 zu einer Insufflationskanüle 30 strömen. Über die Insufflationskanüle 30, bei der es sich bspw. um eine Insufflationsnadel handelt, kann das Insufflationsgas 14 in einen abdominalen Bereich 32 eines Menschen oder Tieres einströmen. Dabei entsteht ein durch das Insufflationsgas 14 aufgeblähter Raum im Bereich 32, so daß dort endoskopische Untersuchungen oder operative Behandlungen mit Hilfe eines schematisch dargestellten Endoskopes 34 durch eine Behandlungsperson 36 möglich sind.

Die Vorrichtung 10 ist zur Befeuchtung des Insufflationsgases 14 ausgestaltet. Dementsprechend enthält die Beladungsvorrichtung 12 eine Befeuchtungsvorrichtung 38, die zur Befeuchtung des Insufflationsgases 14 mit einer Befeuchtungsflüssigkeit 40 dient, die die Arzneimittel 16 und 18 enthält.

Die Aufbereitungskammer 20, die eine Befeuchtungskammer bildet, ist in der Zeichnung stark schematisiert als Hohl-Kubus dargestellt. Im unteren Bereich der Aufbereitungskammer 20 befindet sich ein Befeuchtungsflüssigkeit 40 enthaltendes Befeuchtungsflüssigkeitsbad 41. In der Befeuchtungsflüssigkeit 40 sind die Arzneimittel 16 und 18 gelöst. Das Insufflationsgas 14 strömt im oberen Bereich der Aufbereitungskammer 20 an dem Befeuchtungsflüssigkeitsbad 41 vorbei und nimmt dabei feinste Tröpfchen der Befeuchtungsflüssigkeit 40 und mithin der Arzneimittel 16, 18 mit. Somit wird das Insufflationsgas 14 befeuchtet und mit den Arzneimitteln 16, 18 beladen.

Dieser Effekt wird dadurch verstärkt, daß die Befeuchtungsflüssigkeit 40 durch Heizmittel 42 erwärmt wird. Mit den Heizmitteln 42, die bspw. eine elektrische, vorzugsweise regelbare, Heizung enthalten, ist die Befeuchtungsflüssigkeit 40 derart erwärmbar, daß sie verdampft. Die Befeuchtungsflüssigkeit 40 wird dabei vernebelt. Durch die Beladung mit der erwärmten Befeuchtungsflüssigkeit 40 wird das Insufflationsgas 14 zudem erwärmt und an das im abdominalen Bereich 32 vorhandene Mikroklima optimal angepaßt. Die vorteilhaft vorhandene Heizung 26 sorgt sozusagen für eine Nacherwärmung des Insufflationsgases 14, die jedoch nicht unbedingt notwendig ist.

Bei dem Insufflationsgas 14 handelt es sich bspw. um Kohlensäuregas (CO₂). Das Arzneimittel 16 ist bspw. ein antibakterielles Arzneimittel, das Arzneimittel 18 ein entzündungshemmendes Arzneimittel. Die beiden Arzneimittel 16, 18 sind jeweils in der Befeuchtungsflüssigkeit 40 gelöst, bei der es sich z.B. um steriles Wasser handelt. "Gelöst" muß hier in einem sehr weiten Sinne verstanden werden, da die Arzneimittel 16, 18 bspw. auch in Form von Emulsionen oder Aufschlämmungen in der Befeuchtungsflüssigkeit 40 enthalten sein können.

Für das Insufflationsgas 14 sowie die jeweils in Befeuchtungsflüssigkeit 40 gelösten Arzneimittel 16 und 18 sind jeweils Vorratsbehälter 44, 46, 48 vorgesehen, die über Leitungen 50, 52 bzw. 54 mit der Aufbereitungskammer 20 verbunden sind (Die erfindungsgemäße Dosiervorrichtung zur dosierbaren Zufuhr des Arzneimittels zu der Befeuchtungsflüssigkeit ist nicht dargestellt). An den Leitungen 50, 52, 54 sind Dosiervorrichtungen 56, 58, 60 angeordnet, mit denen der Durchfluß des Insufflationsgases 14 bzw. der Befeuchtungsflüssigkeit 40 mit den Arzneimitteln 16 und 18 einstellbar ist. Bei den Dosiervorrichtungen 56 bis 60 handelt es sich bspw. um Ventilanordnungen. Die Dosiervorrichtung 56 enthält z.B. eine Druckregelung, Sicherheitsventile, Entlüftungsventile oder dergleichen. Die Dosiervorrichtung 56 dient zur Druckminderung und Druckeinstellung des unter hohem Druck von ca. 5-60 bar im Vorratsbehälter 44 bereitstehenden Insufflationsgases 14. Die Leitung 50 mündet mit dem Einlaß 22 im oberen Bereich der Aufbereitungskammer 20, die beiden Leitungen 52 und 54 münden im unteren Bereich der Aufbereitungskammer 20.

An dieser Stelle sei angemerkt, daß die Aufbereitung des Insufflationsgases 14, bei der Vorrichtung 10 die Befeuchtung und Beladung mit den Arzneimitteln 16 und 18, vorzugsweise niederdruckseitig erfolgt. Dementsprechend ist die druckmindernde Dosiervorrichtung 56 der Aufbereitungskammer 20 vorgeschaltet. Weiter ist es bevorzugt, daß die Aufbereitung des Insufflationsgases 14 möglichst nahe am Ausgang der Vorrichtung 10 erfolgt. Daher befindet sich die Aufbereitungskammer 20 nahe an der Leitung 28.

Die Vorratsbehälter 44, 46, 48 sind in der Zeichnung gleichartig und stark vereinfacht dargestellt. Sie sind auswechselbar an der Beladungsvorrichtung 12 angeordnet. Prinzipiell wäre es aber auch möglich, statt der auswechselbaren Vorratsbehälter 44 bis 48 mit der Vorrichtung 10 fest verbundene, nachfüllbare Vorratskammern vorzusehen. Beim Ausführungsbeispiel sind die Vorratsbehälter 44, 46, 48 Kartuschen. An den Leitungen 50, 52, 54 sind jeweils Anschlußstücke 62, 64, 66 angeordnet, an denen die Vorratsbehälter 44, 46, 48 mit Anschlußgegenstücken 68, 70, 72 befestigbar sind. Die Anschlußstücke 62, 64, 66 bilden zusammen mit den dazu passenden Anschlußgegenstücken 68, 70, 72 bspw. Bajonett- oder Schraubverbindungen. Ein entleerter Vorratsbehälter 44, 46, 48 kann leicht gegen einen vollen Vorratsbehälter ausgetauscht werden. Die Anschlußstücke 62, 64, 66 können jeweils individuell kodiert sein, um ein fehlerhaftes Anschließen eines Vorratsbehälters 44, 46, 48 mit ungeeignetem Inhalt zu vermeiden.

Wie bereits erläutert, können die Arzneimittel 16, 18 in Form von Emulsionen oder Aufschlämmungen in der Befeuchtungsflüssigkeit 40 enthalten sein. An der Aufbereitungskammer 20 angeordnete Verteilmittel 74 verteilen die Arzneimittel 16, 18 gleichmäßig in der Befeuchtungsflüssigkeit 40 und verhindern somit eine Sedimentbildung. Die Verteilmittel 74 enthalten eine Rührvorrichtung 76 sowie eine Umwälzpumpe 78.

Die Rührvorrichtung 76 weist einen im unteren Bereich der Aufbereitungskammer 20 angeordneten Rühr-Propeller 80 auf, der von einem Elektromotor 82 mittels einer Welle 84 angetrieben wird.

Die Umwälzpumpe 78 ist über Leitungen 86 und 88 mit dem unteren Bereich der Aufbereitungskammer 20 verbunden. Über die Leitung 86 saugt die Umwälzpumpe 78 bspw. die Befeuchtungsflüssigkeit 40 an und pumpt diese über die Leitung 88 wieder in die Aufbereitungskammer 20 zurück. Dadurch entsteht innerhalb der Aufbereitungskammer 20 eine Strömung, die zu einer feinen Verteilung der Arzneimittel 16, 18 in der Befeuchtungsflüssigkeit 40 führt.

Es versteht sich, daß die Verteilmittel 74 lediglich die Rührvorrichtung 76 oder die Umwälzpumpe 78 enthalten könnten. Ferner sind auch andere Ausführungsformen bei den Verteilmitteln 74 möglich. Bspw. könnten sie einen Schieber enthalten, der im unteren Bereich der Aufbereitungskammer 20 hin- und herbewegt wird.

Die Funktionen der Vorrichtung 10 werden von einer zentralen Steuereinheit 90 gesteuert und überwacht. Die Steuereinheit 90 bildet zum einen ein Gas-Steuermittel zur Steuerung der Menge und des Drucks des dem Auslaß 24 zugeführten Insufflationsgases 14 und zum anderen ein Dosiersteuermittel zur Dosierung des Anteils der Befeuchtungsflüssigkeit 40 sowie der Arzneimittel 16 und 18 in dem Insufflationsgas 14.

Bei der Steuereinheit 90 handelt es sich bspw. um eine Mikroprozessorsteuerung mit einem Mikroprozessor 92. Die Steuereinheit 90 weist Ausgabemittel 94, bspw. ein Display und einen Lautsprecher, sowie Eingabemittel 96, bspw. Bedientasten, auf. Über Steuerleitungen 98, 100, 102, 104, 106 steuert und überwacht die Steuereinheit 90 die Heizmittel 42, die Rührvorrichtung 76, die Umwälzpumpe 78 sowie die Dosiervorrichtungen 56, 58 bzw. 60. Ferner steuert die Steuereinheit 90 über eine Steuerleitung 110 die Heizung 26.

Zur Bildung der über die Steuerleitungen 98 bis 110 versendeten Steuersignale sind Melde-Informationen, z.B. über Druck, Temperatur, Feuchtigkeitszustand, Füllstand oder dergleichen erforderlich. Zur Erzeugung und Übermittlung derartiger Melde-Informationen sind Sensoren vorgesehen, von denen beispielhaft Sensoren 112 und 114 gezeigt sind.

Der Sensor 112 ist am Auslaß 24 angeordnet und meldet der Steuereinheit 90, in welchem Maße das Insufflationsgas 14 mit Befeuchtungsflüssigkeit 40 bzw. mit den Arzneimitteln 16, 18 gesättigt ist. Dementsprechend kann die Steuereinheit 90 bspw. die Heizmittel 42 zur stärkeren oder geringeren Erwärmung der Befeuchtungsflüssigkeit 40 ansteuern.

Der Sensor 114 ist im unteren Bereich der Aufbereitungskammer 20 angeordnet und meldet der Steuereinheit 90 bspw. den Anteil des Arzneimittels 16 in der Befeuchtungsflüssigkeit 40, die sich in der Aufbereitungskammer 20 befindet. Dementsprechend kann die Steuereinheit 90 bspw. die Dosiervorrichtung 58 ansteuern.

Bei dem oben im Zusammenhang mit der Vorrichtung 10 erläuterten Ausführungsbeispiel wird die Befeuchtungsflüssigkeit 40 erwärmt, was zum einen zu einem gewissen Verdampfungseffekt der Befeuchtungsflüssigkeit 40 führt und zum anderen eine optimale Anpassung des Insufflationsgases 14 an die im abdominalen Bereich 32 herrschenden mikroklimatischen Verhältnisse, also die dortige Temperatur und die dort herrschende Feuchtigkeit, ermöglicht. Allerdings gibt es auch Arzneimittel, die möglichst keiner thermischen Belastung ausgesetzt werden sollten.

Die Variante der Vorrichtung 10 ist in gepunkteten Linien in die Fig. 1 eingezeichnet.

Am Einlaß 22 ist hierbei eine Einblasvorrichtung 116 angeordnet. Die Einblasvorrichtung 116 ist vorliegend ein in Richtung des unteren Bereiches der Aufbereitungskammer 20 gekrümmtes Leitungsstück, das in dem Befeuchtungsflüssigkeitsbad 41 mündet. Das aus dem Vorratsbehälter 44 ausströmende Insufflationsgas 14 wird dementsprechend in die Befeuchtungsflüssigkeit 40 eingeblasen. Wenn das Insufflationsgas 14 aus der Befeuchtungsflüssigkeit 40 wieder austritt, nimmt es geringe Mengen von dieser sowie von den darin enthaltenen Arzneimitteln 16, 18 mit und strömt zum Auslaß 24. Das Insufflationsgas 14 wird sozusagen durch das Befeuchtungsflüssigkeitsbad 41 geführt.

Es ist eine Gas-Abfuhrleitung 29 vorgesehen, über die Gas aus dem abdominalen Bereich 32 wieder abgeführt werden kann und einer Meßvorrichtung 93 zugeführt wird. Die Meßvorrichtung 93 weist einen Drucksensor auf, der den Druck des Gases im abdominalen Bereich mißt. Das vom Sensor erzeugte Signal wird zur Steuerung der zuzuführenden Menge an Insufflationsgas der Dosiervorrichtung 56 zugespeist.

## Patentansprüche

1. Vorrichtung zur Aufbereitung eines Insufflationsgases, mit einem Einlass (22) zum Zuführen eines Insufflationsgases (14), mit einer Befeuchtungsvorrichtung (38) zur Befeuchtung des Insufflationsgases (14) mit einer Befeuchtungsflüssigkeit (40), die ein Arzneimittel (16, 18) enthält, und mit einem Auslass (24) zum Abführen des mit dem Arzneimittel (16, 18) beladenen Insufflationsgases (14), wobei die Befeuchtungsvorrichtung (38) eine Aufbereitungskammer (20) aufweist und wobei die Aufbereitungskammer (20) zumindest eine Leitung (52, 54) zur Zufuhr der Befeuchtungsflüssigkeit (40) mit dem darin enthaltenen Arzneimittel (16, 18) aufweist, **dadurch gekennzeichnet, dass** die Befeuchtungsvorrichtung (38) eine Einblasvorrichtung (116) zum Einblasen des Insufflationsgases (14) in die das Arzneimittel (16, 18) enthaltende Befeuchtungsflüssigkeit (40) aufweist, aus der das Insufflationsgas (14) zu dem Auslass (24) hin unter Mitnahme von das Arzneimittel (16, 18) enthaltender Befeuchtungsflüssigkeit (40) austreten kann, und dass sie eine Dosiervorrichtung zur dosierbaren Zufuhr des Arzneimittels (16, 18) zu der Befeuchtungsflüssigkeit (40) aufweist.

2. Vorrichtung nach Anspruch 1, **dadurch gekennzeichnet, dass** sie Dosier-Steuermittel (90, 112) zur Dosierung des Anteils der das Arzneimittel (16, 18) enthaltenden Befeuchtungsflüssigkeit (40) in dem Insufflationsgas (14) aufweist.

3. Vorrichtung nach Anspruch 1 oder 2, **dadurch gekennzeichnet, dass** sie Verteilmittel (74) zur Verteilung des Arzneimittels (16, 18) in der Befeuchtungsflüssigkeit (40) aufweist.

4. Vorrichtung nach Anspruch 3, **dadurch gekennzeichnet, dass** die Verteilmittel (74) eine Rührvorrichtung (76) zum Rühren der Befeuchtungsflüssigkeit (40) enthalten.

5. Vorrichtung nach Anspruch 3, **dadurch gekennzeichnet, dass** die Verteilmittel (74) eine Umwälzpumpe (78) zum Umwälzen der Befeuchtungsflüssigkeit (40) und des Arzneimittels (16, 18) enthalten.

6. Vorrichtung nach einem der Ansprüche 1 bis 5, **dadurch gekennzeichnet, dass** sie Temperiermittel zum Temperieren der Befeuchtungsflüssigkeit (40) aufweist.

7. Vorrichtung nach Anspruch 6, **dadurch gekennzeichnet, dass** die Temperiermittel Heizmittel (42) zum Verdampfen der Befeuchtungsflüssigkeit (40) enthalten.

## Claims

1. Device for conditioning an insufflation gas with an inlet (22) for delivering an insufflation gas (14),
with a humidifying device (38) for humidifying the insufflation gas (14) with a humidifying liquid (40) containing a medicament (16, 18), and
with an outlet (24) for withdrawing the insufflation gas (14) charged with the medicament (16, 18),
whereby the humidifying device (38) comprises a conditioning chamber (20), and
whereby the conditioning chamber (20) comprises at least one line (52, 54) for the delivery of the humidifying liquid (40) with the medicament (16, 18) contained therein,
**characterized in that** the humidifying device (38) comprises a blower device (116) for blowing the insufflation gas (14) into the humidifying liquid (40) containing the medicament (76, 18) from which the insufflation gas (14) can exit towards the outlet (24) while picking up the humidifying liquid (40) containing the medicament (16, 18) and that it comprises a dosing device for a dosed delivery of the medicament (16, 18) to the humidifying liquid (40).

2. Device according to claim 1, **characterized in that** it comprises dose control means (90, 112) for dosing the proportion of the humidifying liquid (40) containing the medicament (16, 18) in the insufflation gas (14).

3. Device according to claim 1 or 2, **characterized in that** it comprises distributing means (74) for distributing the medicament (16, 18) in the humidifying liquid (40).

4. Device according to claim 3, **characterized in that** the distributing means (74) comprise a stirring device (76) for stirring the humidifying liquid (40).

5. Device according to claim 3, **characterized in that** the distributing means (74) comprise a circulating pump (78) for circulating the humidifying liquid (40) and the medicament (16, 18).

6. Device according to any one of claims 1 to 5, **characterized in that** it comprises temperature control means for controlling the temperature of the humidifying liquid (40).

7. Device according to claim 6, **characterized in that** the temperature control means comprise heating means (42) for evaporating the humidifying liquid (40).

## Revendications

1. Dispositif de préparation d'un gaz d'insufflation, comportant une entrée (22) pour amener un gaz d'insufflation (14), un dispositif d'humidification (38) pour humidifier le gaz d'insufflation (14) avec un liquide d'humidification (40) qui contient un médicament (16, 18), et une sortie (24) pour extraire le gaz d'insufflation (14) chargé avec le médicament (16, 18), le dispositif d'humidification (38) présentant une chambre de préparation (20) et la chambre de préparation (20) présentait au moins une conduite (52, 54) pour amener le liquide d'humidification (40) avec le médicament (16, 18) qu'il contient, **caractérisé en ce que** le dispositif d'humidification (38) présente un dispositif d'insufflation (116) pour insuffler le gaz d'insufflation (14) dans le liquide d'humidification (40) contenant le médicament (16, 18), duquel le gaz d'insufflation (14) peut s'échapper vers la sortie (24) en entraînant le liquide d'humidification (40) contenant le médicament (16, 18), et qu'il présente un dispositif de dosage pour amener de manière dosable le médicament (16, 18) au liquide d'humidification (40).

2. Dispositif selon la revendication 1, **caractérisé en ce qu'**il présente des moyens de commande de dosage (90, 112) pour doser la proportion de liquide d'humidification (40) contenant le médicament (16, 18) dans le gaz d'insufflation (14).

3. Dispositif selon la revendication 1 ou 2, **caractérisé en ce qu'**il présente des moyens de répartition (74) pour répartir le médicament (16, 18) dans le liquide d'humidification (40).

4. Dispositif selon la revendication 3, **caractérisé en ce que** les moyens de répartition (74) comprennent un dispositif mélangeur (76) pour mélanger le liquide d'humidification (40).

5. Dispositif selon la revendication 3, **caractérisé en ce que** les moyens de répartition (74) comprennent une pompe de circulation (78) pour faire circuler le liquide d'humidification (40) et le médicament (16, 18).

6. Dispositif selon l'une des revendications 1 à 5, **caractérisé en ce qu'**il présente des moyens de régulation de la température pour réguler la température du liquide d'humidification (40).

7. Dispositif selon la revendication 6, **caractérisé en ce que** les moyens de régulation de la température comprennent des moyens de chauffage (42) pour vaporiser le liquide d'humidification (40).
